# EUROPEAN PATENT APPLICATION

(11) **EP 4 667 902 A1**
(43) Date of publication of application: **24.12.2025**
(21) Application number: 23922317.5
(22) Date of filing: 07.10.2023
(51) Int. Cl.: G01N 15/14, G06T 7/00, G01N 33/68

(54) **IMAGING FLOW CYTOMETRY-BASED HIGH-THROUGHPUT DRUG SCREENING METHOD**

(30) Priority: 14.02.2023 CN 202310114892
(71) Applicant: Fairy Life Sciences (Wuhan) Co., Ltd., Wuhan, Hubei 430075 (CN)
(72) Inventor: ZHAO, Jingjing, Zhengzhou, Henan 450046 (CN); DU, Lin, Zhengzhou, Henan 450046 (CN)
(74) Representative: Metida
(86) International application number: PCT/CN2023/123195
(87) International publication number: WO 2024/169199

(57) **Abstract**

An imaging flow cytometry-based high-throughput drug screening method, comprising: cell incubation, cell staining, acquiring single-cell images by means of a flow cytometer, and image extraction and analysis, which combines the high-throughput advantages of flow cytometry and the imaging capability of a microscope, so that multi-channel single-cell images can be generated in a high throughput manner, thereby implementing acquisition of fluorescent images and unmarked images of single cells at a throughput of 102-105 cells per second, which can be used for cell phenotype drug screening and improving the screening efficiency by a factor of 102-104.

## Description

### CROSS-REFERENCE TO RELATED APPLICATIONS

The present application claims priority to Chinese Patent Application No. 202310114892.7, filed on February 14, 2023, the entire contents of which are incorporated herein by reference.

### TECHNICAL FIELD

The present invention relates to the field of instrument analysis technology, and in particular to an imaging flow cytometry-based high-throughput drug screening method.

### BACKGROUND

The common method for drug screening is cell-based phenotypic screening. The existing cell-based phenotypic drug screening protocols generally perform preliminary high-throughput screening first by flow cytometry, and then complete precise analysis by high-resolution fluorescence microscopy. Flow cytometers can achieve the high-throughput detection of biological particles at a throughput up to a range of 10³-10⁵ particles per second, and detect scattered light and fluorescence signals of biological particles. Meanwhile, flow cytometers have high parallel detection capabilities. For example, the parallel detection capabilities of the most advanced spectral flow cytometer can be embodied by 64 fluorescent channels, while conventional flow cytometers have 4-14 fluorescent channels as well as one forward scattered light channel and one side scattered light channel. However, flow cytometers do not have two-dimensional spatial resolution or imaging capabilities, and cannot acquire morphological information of cells. As such, in the existing cell-based phenotypic drug screening protocols, precise analysis must be completed with the help of high-resolution fluorescence microscopy, but the number of cells in a single imaging field of view of a fluorescence microscope is extremely limited, namely dozens of cells per field of view in general, which limits the speed of cell-based phenotypic drug screening. Therefore, there is an urgent need to provide a high-throughput drug screening method.

### SUMMARY OF THE INVENTION

The present invention is to at least solve one of the technical problems in the relevant art to some extent. To this end, one purpose of the present invention is to provide an imaging flow cytometry-based high-throughput drug screening method, specifically comprising treating cells with candidate drugs, performing fluorescent labeling, acquiring single-cell images by a flow cytometer, and extracting and analyzing the single-cell images.

As such, the present invention provides a high-throughput drug screening method, comprising:
(1) treating cells to be detected with various candidate drugs, respectively;
(2) performing fluorescent labeling of the candidate drug-treated cells that are obtained in step (1) to prepare a cell suspension;
(3) placing the cell suspension obtained in step (2) in flow channels of an imaging flow cytometer for detection to obtain single-cell images; and
(4) extracting information from the single-cell images obtained in step (3) and acquiring analysis results based on the extracted information through an AI algorithm to screen out drugs that meet predetermined requirements.

Imaging flow cytometer relates to newly developed techniques for analysis of biological particles in recent years. By combining flow technology and high-speed microscopy technology, it realizes multi-channel fluorescence and scattered light imaging of biological particles in a high-speed flow state, and has the high-throughput characteristics of conventional flow cytometers and the two-dimensional spatial resolution capability of microscopic imaging. The present invention can generate multi-channel single-cell images in a high-throughput manner by using imaging flow cytometry for cell-based phenotypic drug screening, thereby increasing the screening efficiency by 10²-10⁴ times.

According to the embodiments of the present invention, the fluorescent labeling in step (2) comprises non-distinctive labeling and/or specific labeling;
wherein the non-distinctive labeling comprises using a uniform fluorescent labeling protocol for the cells treated with the candidate drugs; and
the specific labeling comprises selective fluorescent labeling of the cells treated with the candidate drugs according to phenotypic differences set by prior knowledge.

According to the embodiments of the present invention, the uniform fluorescent labeling protocol comprises uniformly labeling cell nucleus, nucleolus, endoplasmic reticulum, Golgi apparatus, cell membrane, and mitochondria of all cells treated with the candidate drugs with different fluorescent dyes.

According to the embodiments of the present invention, the selective fluorescent labeling according to phenotypic differences set by prior knowledge comprises fluorescent labeling of specific proteins on the cell surface or inside cells.

The present invention can fluorescently label cell components and organelles (e.g., cell membrane, cell nucleus, endoplasmic reticulum, Golgi apparatus, nucleolus, actin, mitochondria), thereby realizing the identification of cell components and organelles. Specifically, the label-free images obtained by non-distinctive labeling comprise a bright field, a dark field and a scattered field, and can measure the overall morphology, size and granularity of cells; through specific labeling, such as fluorescent labeling of certain cell components or organelles, their fluorescent images are available, and the corresponding image information are further available.

According to the embodiments of the present invention, the single-cell images in step (3) include fluorescent images and label-free images of cells.

According to the embodiments of the present invention, the information extracted from the single-cell images in step (4) comprises at least one selected from the group consisting of an organelle, a cell component, genetic material, cell morphology, organelle distribution, protein expression, nuclear size, cell morphological information, and cell texture.

According to the embodiments of the present invention, the analysis results comprise at least one selected from the group consisting of cell proliferation, cell apoptosis, protein expression of cells, cell health, drug toxicity to non-target cells, and drug killing effect on target cells.

The information extracted from the images by the present invention comprises an organelle, a cell component, genetic material, cell morphology, organelle distribution, protein expression, nuclear size, cell morphological information, cell texture, and other information. The corresponding analysis involves various aspects of cells, such as cell proliferation, cell apoptosis, protein expression of cells, cell health, drug toxicity to non-target cells, drug killing effect on target cells, and so on.

According to the embodiments of the present invention, the liquid flow in the flow channels of the imaging flow cytometer in step (3) has a central flow velocity of 0.1-20 m/s.

According to the embodiments of the present invention, acquiring analysis results based on the extracted information through an AI algorithm is achieved by either of the following methods, to screen out drugs that meet predetermined requirements:
Method 1: (1) constructing a data set using the extracted information, and learning the data set by an AI algorithm to acquire the morphology, spatial distribution and expression of the organelle and molecular information in the single-cell images; and
(2) performing qualitative and quantitative analysis on the morphology, spatial distribution and expression of the organelle and molecular information obtained in step (1), and setting preset conditions to automatically screen out drugs that meet the predetermined requirements;
or Method 2: (A) automatically clustering the cells to be detected after analyzing the extracted information by an AI algorithm; and
(B) screening out drugs that meet the predetermined requirements after manual analysis based on the clustering results acquired in step (A) and the corresponding single-cell images.

Additional aspects and advantages of the present invention will be set forth in part in the description which follows, and in part will be evident from the description which follows, or will be learned by practice of the present invention.

### BRIEF DESCRIPTION OF THE DRAWINGS

The above and/or additional aspects and advantages of the present invention will become apparent and easily understood with reference to the following description of the embodiments made in the drawings, in which:
FIG. 1 shows the imaging flow cytometry-based high-throughput drug screening process of the present invention;
FIG. 2 shows that Example 1 of the present invention determines the blocking effect of drugs on the proliferation of cancer cells by analyzing cell cycle images;
FIG. 3 shows the imaging flow cytometry-based process of establishing combinations of phenotypic data in Example 2 of the present invention;
FIG. 4 shows the process of screening optimal drugs based on the combinations of phenotypic data and evaluation algorithm in Example 2 of the present invention.

### DETAILED DESCRIPTION

Hereinafter, embodiments of the present invention will be described in detail. The embodiments described below are exemplary and are only used to explain the present invention, and shall not be construed as limitations to the present invention.

It should be noted that the terms "first" and "second" are used merely for the purpose of description, and shall not be construed as indicating or implying relative importance or implicitly indicating a quantity of indicated technical features. Thus, the features defined with "first" and "second" may explicitly or implicitly include one or more of the features. Further, in the description of the present invention, "more" indicates two or above unless otherwise specified.

The endpoints and any values of the ranges disclosed herein are not limited to the exact ranges or values, and these ranges or values should be understood to include values close to these ranges or values. As for numerical ranges, the endpoint values of the various ranges, the endpoint values and the individual point value of the various ranges, and the individual point values may be combined with one another to produce one or more new numerical ranges, which should be regarded as having been specifically disclosed herein.

In order to make the present invention more easily understood, certain technical and scientific terms are specifically defined below. Unless specifically defined elsewhere in this document, all other technical and scientific terms used herein have the meaning as commonly understood by one of ordinary skill in the art to which the present invention belongs.

Herein the terms "include" or "comprise" are open-ended expressions, that is, including the contents specified in the present invention but not excluding other contents.

According to one specific embodiment of the present invention, the present invention provides a high-throughput drug screening method, comprising:
(1) treating cells to be detected with various candidate drugs, respectively;
(2) performing fluorescent labeling of the candidate drug-treated cells that are obtained in step (1) to prepare a cell suspension;
(3) placing the cell suspension obtained in step (2) in flow channels of an imaging flow cytometer for detection to obtain single-cell images; and
(4) extracting information from the single-cell images obtained in step (3) and acquiring analysis results based on the extracted information through an AI algorithm to screen out drugs that meet predetermined requirements.

According to one specific embodiment of the present invention, the cells to be detected in the step of "treating the cells to be detected with various candidate drugs" may be cells of the same type or of different types. Taking lung cancer cells as an example, various candidate drugs can be used to treat lung cancer cells to screen out a drug that meets the requirements; meanwhile, the selected drug is applied to different cells to determine the specificity of the drugs, and to lung cancer cells and healthy cells to determine the toxicity of the drug to healthy cells. The present invention can achieve high-throughput drug screening based on an imaging flow cytometer. It can not only screen multiple candidate drugs for the same cell to obtain a suitable drug, but also screen multiple candidate drugs for different cell types at the same time to obtain suitable drugs for different cell types. According to one specific embodiment of the present invention, the fluorescent labeling includes non-distinctive labeling and/or specific labeling;
wherein the non-distinctive labeling comprises using a uniform fluorescent labeling protocol for the cells treated with the candidate drugs. The uniform fluorescent labeling protocol comprises uniformly labeling cell nucleus, nucleolus, endoplasmic reticulum, Golgi apparatus, cell membrane, and mitochondria of all cells treated with the candidate drugs with different fluorescent dyes. It should be noted that the components and organelles labeled for cells in the uniform fluorescent labeling protocol are not limited to those listed above, and may also be other components and organelle types of cells.

According to one specific embodiment of the present invention, the specific labeling comprises selective fluorescent labeling of the cells treated with the candidate drug according to predicted phenotypic differences.

According to one specific embodiment of the present invention, the single-cell images in step (3) include fluorescent images and label-free images of cells.

According to one specific embodiment of the present invention, the label-free images comprise a bright field, a dark field and a scattered field, and can measure the overall morphology, size and granularity of cells.

According to one specific embodiment of the present invention, the information extracted from the single-cell images in step (4) comprises at least one selected from the group consisting of an organelle, a cell component, genetic material, cell morphology, organelle distribution, protein expression, nuclear size, cell morphological information, and cell texture. It should be noted that the information extracted from the single-cell images in the present invention includes, but is not limited to, the above information. This extracted information constitutes a quantitative information library, and the corresponding relationship between the quantitative information library and the analysis results is established through an AI algorithm.

According to one specific embodiment of the present invention, the analysis results comprise at least one selected from the group consisting of cell proliferation, cell apoptosis, protein expression of cells, cell health, drug toxicity to non-target cells, and drug killing effect on target cells.

According to one specific embodiment of the present invention, the liquid flow in the flow channels of the imaging flow cytometer in step (3) has a central flow velocity of 0.1-20 m/s. The central flow velocity of the liquid flow can be adjusted properly according to the needs, and shall not be limited to 0.1-20 m/s.

According to one specific embodiment of the present invention, acquiring analysis results based on the extracted information through an AI algorithm is achieved by either of the following methods, to screen out drugs that meet predetermined requirements:
Method 1: (1) constructing a data set using the extracted information, and learning the data set by an AI algorithm to acquire the morphology, spatial distribution and expression of the organelle and molecular information in the single-cell images; and
(2) performing qualitative and quantitative analysis on the morphology, spatial distribution and expression of the organelle and molecular information acquired in step (1), and automatically screening out drugs that meet the predetermined requirements according to preset conditions;
or Method 2: (A) automatically clustering the cells to be detected after analyzing the extracted information by an AI algorithm; and
(B) screening out drugs that meet the predetermined requirements after manual analysis based on the clustering results acquired in step (A) and the corresponding single-cell images.

Specifically, Method 2 is to obtain analysis results based on the extracted information through an AI algorithm in the absence of a previous data set. The AI algorithm may be t-distributed stochastic neighbor embedding, that is, a dimensionality reduction algorithm with t-SNE, or other algorithms for exploring high-dimensional data.

The solutions of the present disclosure will be explained in conjunction with the examples below. Those skilled in the art will understand that the following examples are only used to illustrate the present disclosure and should not be regarded as limiting the scope of the present disclosure. If no specific techniques or conditions are specified in the examples, the techniques or conditions described in literatures of the art or the product instructions shall be followed. The reagents and instruments in use for which the manufacturers are not specified are all conventional products that can be purchased commercially.

### EXAMPLES

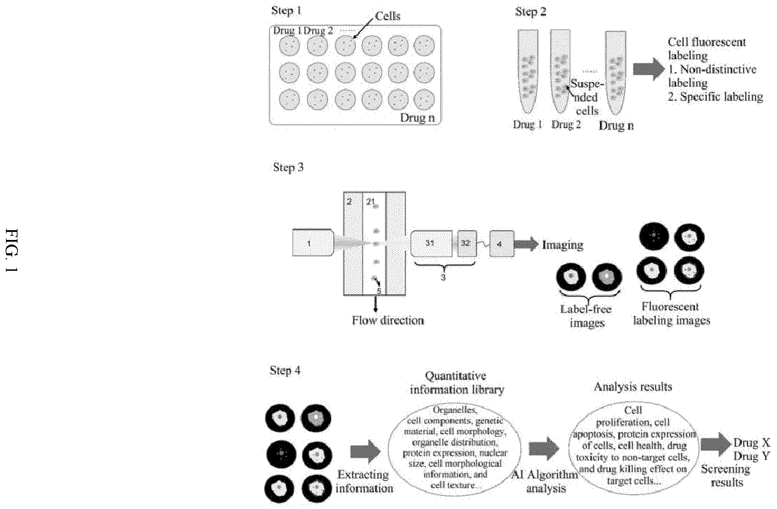

FIG. 1 shows the specific steps of an imaging flow cytometry-based high-throughput drug screening method, comprising:
Step 1: At the cell incubation stage, it is required to add various drugs, thereby observing the phenotypic changes of cells under the action of different candidate drugs;
Step 2: The candidate drug-treated cells that are obtained in step (1) are fluorescently labeled to prepare a cell suspension; wherein the fluorescent labeling is classified into two categories: one category is non-distinctive labeling, i.e., all cells are subjected to an uniform fluorescent labeling protocol, for example, uniformly labeling cell nucleus, nucleolus, endoplasmic reticulum, Golgi apparatus, cell membrane, and mitochondria of all the candidate drug-treated cells with different fluorescent dyes; and the other category is specific labeling, where different cells are subjected to specific fluorescent labeling based on possible phenotypic differences, for example, fluorescently labeling some expressed proteins of cells;
Step 3: The cell suspension obtained in step 2 is placed in flow channels of an imaging flow cytometer for detection to obtain single-cell images. The imaging flow cytometer mainly comprises a laser illumination unit 1, a flow chamber 2, an imaging detection unit 3, and a signal processing unit 4. The laser illumination unit 1 is responsible for shaping one or more laser beams into the required illumination spot. The flow chamber 2 is responsible for focusing suspended cells 5 at the center of flow channels 21 to ensure that the suspended cells 5 can be illuminated by the illumination spot and transmit scattered light and fluorescence. The liquid flow in the flow channels 21 of the flow chamber has a central flow velocity in a range of 0.1-20 m/s, at a throughput of 10²-10⁵ cells per second. An optical system 31 in the imaging detection unit 3 is responsible for collecting the scattered light and fluorescence of each particle, and a photoelectric conversion device 32 therein is responsible for converting the optical signal into an electrical signal. The signal processing unit 4 is responsible for processing the collected signal and outputting a scattered light image and a fluorescence image, wherein the scattered light image comprises a bright field, a dark field and a side scattering image, and the fluorescence image comprises 1-50 fluorescent channel signals. The single-cell images obtained in this step include fluorescent labeling images and label-free images. The fluorescent labeling images can be used to obtain the image information of various cell components and organelles (such as cell membrane, cell nucleus, endoplasmic reticulum, Golgi apparatus, nucleolus, actin, mitochondria, and expression proteins); the label-free images comprise a bright field, a dark field and a scattered field, and can measure the overall morphology, size and granularity of cells;
Step 4: The single-cell images obtained in step 3 are subjected to information extraction and analysis. The information extracted from the images comprises an organelle, a cell component, genetic material, cell morphology, organelle distribution, protein expression, nuclear size, cell morphological information, cell texture and other contents. The corresponding analysis involves different aspects of cells, such as cell proliferation, cell apoptosis, protein expression of cells, cell health, drug toxicity to non-target cells, drug killing effect on target cells, and so on. This step should extract as much imaging information as possible and analyze it through artificial intelligence technology, design a screening protocol based on the measurement data, and select candidate drugs that meet the requirements.

### EXAMPLE 1

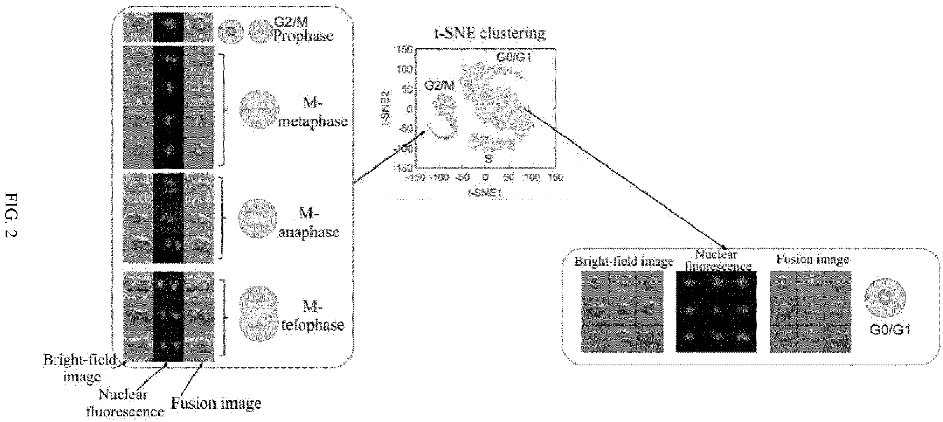

Drugs that block the proliferation of cancer cells are studied. The effects of various candidate drugs on the proliferation of cancer cells and healthy tissue cells are observed. An equal number of healthy tissue cells and cancer cells are taken and cultured in a suitable environment after various candidate drugs are added, and control groups with no addition of any drugs are set up. After a period of culture, such as 5 to 9 days, the cultured cells are prepared into a single-cell suspension, and the cell nuclei are stained with SYTO16. Subsequently, each single cell sample is imaged through an imaging flow system to collect bright-field and nucleus fluorescence images of the cells. As shown in FIG. 2, according to the morphology of cell nuclei and the cell contour profiles in the bright field, an AI algorithm can distinguish the G0/G1, S, G2, and M stages in the cell cycle. The healthy tissue cells with no addition of any drugs are taken as the control group A, and the number and relative proportion of cells in different cell cycle stages are counted. The cancer cells with no addition of any drugs are taken as the control group B, and the number and relative proportion of cells in different cell cycle stages are counted. Since cancer cells proliferate faster than healthy cells, cancer cells after culture have a greater absolute number than healthy tissue cells, and cells in the division phase are in a higher proportion. The images and statistical data of candidate drug C on healthy tissue cells and cancer cells are observed. If the images and statistical data of candidate drug C on healthy tissue cells are close to those of the control group A, it is indicated that candidate drug C is not toxic to healthy cells; if abnormal, such as a decrease in the absolute number of cells and/or a decrease in the proportion of cells in the division phase, it is indicated that candidate drug C has toxic side effects on healthy tissue cells. It is necessary to further study the effects of candidate drug C on healthy cells or exclude it from the candidate list. If the images and statistical data of candidate drug C on cancer cells are close to those of the control group B, it is indicated that candidate drug C cannot effectively block the proliferation of cancer cells and is not a desired candidate drug; if the absolute number of cancer cells upon the action of candidate drug C decreases significantly, and a number of cancer cells are blocked in one of the G0/G1, S, G2, and M stages, it is indicated that candidate drug C can effectively block the proliferation of cancer cells, and the drug target can be further analyzed according to the blocking position. Finally, several drugs that can effectively block the proliferation of cancer cells and have no obvious side effects on healthy tissue cells are selected, and these drugs are studied more deeply and extensively to finally determine the optimal drug. Generally, for small-molecule compound drugs, the initial candidate drugs can be as many as thousands to hundreds of thousands, so high-throughput imaging flow analysis plays an irreplaceable role in improving the efficiency of drug screening and accelerating drug development.

### EXAMPLE 2

Drug screening for against viral infections in the lungs is studied. The cell model adopts MRC-5 human lung fibroblasts, the virus to be studied is human coronavirus CoV-229E, and the candidate drugs include those coded as Drug 1, Drug 2, ..., and Drug 100. The staining reagents in use are Hoechst 33342 for DNA staining (an excitation wavelength of 377 nm, an emission wavelength of 447 nm), a wheat germ agglutinin-Alexa Fluor 555 conjugate for the staining of Golgi apparatus and cell membrane (an excitation wavelength of 562 nm, an emission wavelength of 624 nm), a phalloidin-Alexa Fluor 568 conjugate for the staining of fibrous actin (an excitation wavelength of 578 nm, an emission wavelength of 603 nm), a SYTO14 green fluorescent dye for the staining of nucleolar and cytoplasmic RNAs (an excitation wavelength of 531 nm, an emission wavelength of 593 nm), a concanavalin A-Alexa Fluor 488 conjugate for the staining of endoplasmic reticulum (an excitation wavelength of 482 nm, an emission wavelength of 536 nm), and coronavirus pan monoclonal antibody FIPV3-70 and goat anti-mouse IgG H&L secondary antibody (an excitation wavelength of 628 nm, an emission wavelength of 692 nm) for coupling coronavirus nucleoprotein. This set of reagents is used to determine whether MRC-5 human lung fibroblasts are infected by human coronavirus CoV-229E. If infected, the fluorescence transmitted by the secondary antibody will be observed. If not infected, there will be no fluorescence signal of the secondary antibody. The experiment is divided into two steps: phenotypic data establishment and drug screening. The purpose of the first step of phenotypic data establishment is to establish an effective combination of phenotypic data based on the multidimensional phenotypic data extracted from the images, which can effectively distinguish infected cells from uninfected cells. The second step of drug screening is to judge the changes of infected cells under the action of different drugs based on the combination of extracted phenotypic data, and finally select drugs with low toxicity and capable of effectively curing diseases.

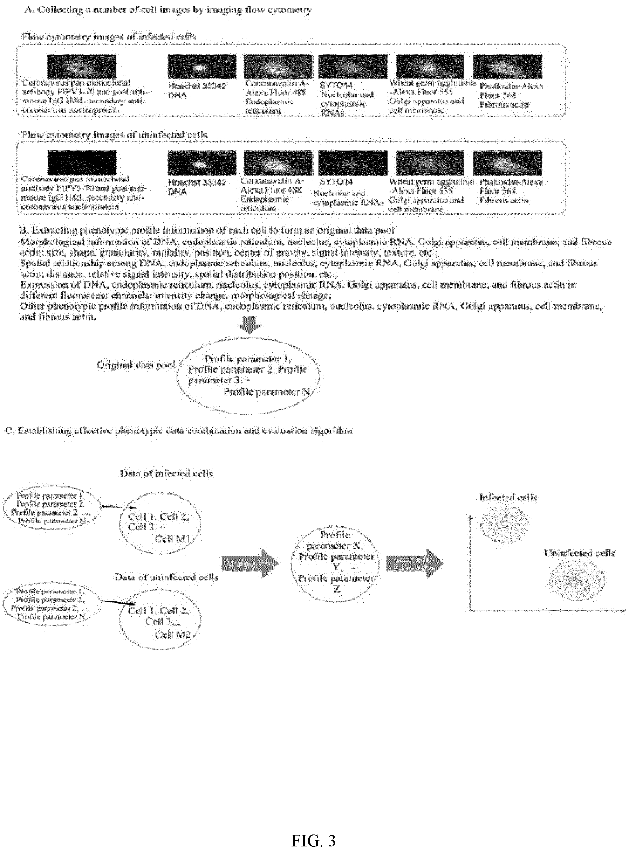

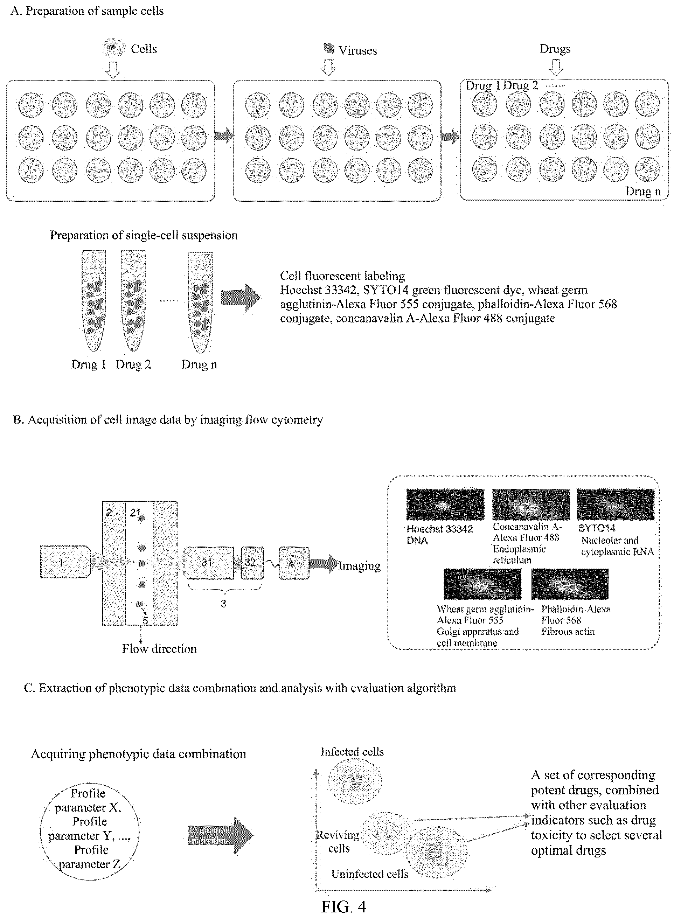

The first step is shown in FIG. 3. First, healthy MRC-5 human lung fibroblasts are infected with human coronavirus CoV-229E, and then different parts of MRC-5 human lung fibroblasts are stained with the above-mentioned Hoechst 33342, the SYTO14 green fluorescent dye, the wheat germ agglutinin-Alexa Fluor 555 conjugate, the phalloidin-Alexa Fluor 568 conjugate, and the concanavalin A-Alexa Fluor 488 conjugate. Then, the coronavirus pan monoclonal antibody FIPV3-70 and goat anti-mouse IgG H&L secondary antibody are added. If MRC-5 human lung fibroblasts are infected with Cov-229E, the coronavirus nucleoprotein will be expressed in the cells and can be labeled. If they are not infected, they will not be labeled. Next, the cells are prepared into a single-cell suspension, and the fluorescent image data and label-free image data of 100,000 cells are quickly collected by imaging flow cytometry. Since the above-mentioned reagents for molecular labeling have different fluorescence wavelengths, the labeled cell structures can be distinguished by different fluorescent channels. Profile information is extracted from each fluorescent image and label-free image, such as the size, shape, granularity, position, center of gravity, texture, and spatial relationship with adjacent cell structures of cells or cell structures, to form an original data pool. Whether a cell is infected is determined by detecting the presence or absence of coronavirus nucleoprotein signals, that is, whether a cell is infected is determined by whether the single cell has a fluorescent signal of a goat anti-mouse IgG H&L secondary antibody. Then, the optimal set of profiles is selected from the original data pool through AI data analysis and an evaluation algorithm is derived. After being processed by the evaluation algorithm, a combination of optimal profiles can most accurately determine whether the cell is infected;
The second step is shown in FIG. 4. Healthy MRC-5 human lung fibroblasts are first assigned to a multi-well plate. After 24 hours of incubation, the cells are infected with human coronavirus CoV-229E. After 2 hours, candidate Drugs 1, Drug 2... and Drug 100 are added to each culture well in turn and these cells are incubated for 48 hours. Next, the cells are fixed, and the cell permeability is increased. The cells are labeled with the above dyes and prepared into a single cell suspension, and then the cell images upon the action of each drug are collected by imaging flow cytometry. The number of cells collected after the action of each drug is not less than 10,000. In this step, the coronavirus pan monoclonal antibody FIPV3-70 and goat anti-mouse IgG H&L secondary antibody are not added. Subsequently, whether the cells are in an infected state is judged according to the combination of optimal profiles and evaluation algorithm determined in the first step. If the phenotypic combination of the cells is consistent with the uninfected state determined in the first step, it is indicated that the drug can effectively resist infection. If the phenotypic combination of the cells is consistent with the infected state determined in the first step, it is indicated that the drug cannot effectively resist human coronavirus CoV-229E. If the phenotypic combination of the cells is closer to the uninfected state, it is indicated that the drug can effectively improve the infected state and help the cells return to a healthy state. Moreover, the toxicity of the drugs to cells can also be determined by observing the images of the cell nuclei. Finally, safe and potent anti-infective drugs are selected from candidate Drug 1, Drug 2, ..., and Drug 100.

In the description of this specification, reference to the terms "one/an example", "some examples", "exemplification", "specific exemplification", "some embodiments" or "some exemplifications" etc. means that the specific features, structures, materials or characteristics described in conjunction with the example or exemplification are included in at least one example or exemplification of the present invention. In this specification, the exemplary expressions of the above terms do not necessarily refer to the same example(s) or exemplification(s). Moreover, the specific features, structures, materials or characteristics described may be combined in any one or more examples or exemplifications in a suitable manner. In addition, those skilled in the art may combine and associate different examples or exemplifications and features in different examples or exemplifications described in this specification without mutual contradiction.

Although the examples of the present invention have been illustrated and described above, it would be appreciated that the above examples are exemplary and cannot be construed as limitations to the present invention. Any changes, modifications, alternatives, and variations can be made by those skilled in the art to the above examples within the scope of the present invention.

## Claims

1. A high-throughput drug screening method, comprising:
(1) treating cells to be detected with various candidate drugs, respectively;
(2) performing fluorescent labeling of the candidate drug-treated cells that are obtained in step (1) to prepare a cell suspension;
(3) placing the cell suspension obtained in step (2) in flow channels of an imaging flow cytometer for detection to obtain single-cell images; and
(4) extracting information from the single-cell images obtained in step (3) and acquiring analysis results based on the extracted information through an AI algorithm to screen out drugs that meet predetermined requirements.

2. The method according to claim 1, wherein the fluorescent labeling in step (2) includes non-distinctive labeling and/or specific labeling;
wherein the non-distinctive labeling comprises using a uniform fluorescent labeling protocol for the cells treated with the candidate drugs; and
the specific labeling comprises selective fluorescent labeling of the cells treated with the candidate drugs according to phenotypic differences set by prior knowledge.

3. The method according to claim 2, wherein the uniform fluorescent labeling protocol comprises uniformly labeling cell nucleus, nucleolus, endoplasmic reticulum, Golgi apparatus, cell membrane, and mitochondria of all the candidate drug-treated cells with different fluorescent dyes respectively.

4. The method according to claim 2, wherein the selective fluorescent labeling according to phenotypic differences set by prior knowledge comprises fluorescent labeling of specific proteins on the cell surface or inside cells.

5. The method according to claim 1, wherein the single-cell images in step (3) include fluorescent images and label-free images of cells.

6. The method according to claim 1, wherein the information extracted from the single-cell images in step (4) comprises at least one selected from the group consisting of an organelle, a cell component, genetic material, cell morphology, organelle distribution, protein expression, nuclear size, cell morphological information, and cell texture.

7. The method according to any one of claims 1-6, wherein the analysis results comprise at least one selected from the group consisting of cell proliferation, cell apoptosis, protein expression of cells, cell health, drug toxicity to non-target cells, and drug killing effect on target cells.

8. The method according to claim 1, wherein a liquid flow in the flow channels of the imaging flow cytometer in step (3) has a central flow velocity of 0.1-20 m/s.

9. The method according to claim 1, wherein acquiring analysis results based on the extracted information through an AI algorithm is achieved by either of the following methods, to screen out drugs that meet predetermined requirements:
Method 1: (1) constructing a data set using the extracted information, and learning the data set by an AI algorithm to acquire the morphology, spatial distribution and expression of the organelle and molecular information in the single-cell images; and
(2) performing qualitative and quantitative analysis on the morphology, spatial distribution and expression of the organelle and molecular information acquired in step (1), and automatically screening out drugs that meet the predetermined requirements according to preset conditions;
or Method 2: (A) automatically clustering the cells to be detected after analyzing the extracted information by an AI algorithm; and
(B) screening out drugs that meet the predetermined requirements after manual analysis based on the clustering results acquired in step (A) and the corresponding single-cell images.
